Europäisches Patentamt
European Patent Office
Office européen des brevets

(19) (11) Veröffentlichungsnummer: **0 037 468**
**A2**

(12)
# EUROPÄISCHE PATENTANMELDUNG

(21) Anmeldenummer: 81101742.5

(22) Anmeldetag: 10.03.81

(51) Int. Cl.³: A 01 N 43/64
A 01 N 47/20, A 01 N 47/12
C 07 D 249/08

(30) Priorität: 24.03.80 DE 3011258

(43) Veröffentlichungstag der Anmeldung: 14.10.81 Patentblatt 81/41

(84) Benannte Vertragsstaaten: AT BE CH DE FR GB IT LI NL SE

(71) Anmelder: BASF Aktiengesellschaft
Carl-Bosch-Strasse 38
D-6700 Ludwigshafen(DE)

(72) Erfinder: Rentzea, Costin, Dr.
Neuenheimer Landstrasse 72
D-6900 Heidelberg(DE)

(72) Erfinder: Ammermann, Eberhard, Dr.
Sachsenstrasse 3
D-6700 Ludwigshafen(DE)

(72) Erfinder: Pommer, Ernst-Heinrich, Dr.
Berliner Platz 7
D-6703 Limburgerhof(DE)

(54) **Fungizide Mittel, enthaltend Triazolylderivate und Verfahren zur Bekämpfung von Pilzen mit ihnen.**

(57) Die vorliegende Anmeldung betrifft fungizide Mittel, enthaltend ein Triazolyderivat der allgemeinen Formel

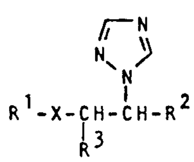

in der $R^1$ und $R^2$ unabhängig voneinander Alkyl, Naphthyl oder gegebenenfalls substituiertes Phenyl bedeuten, und $R^3$ Alkyl, gegebenenfalls substituiertes Benzyl oder gegebenenfalls substituiertes Phenyl bedeutet, und X für C=O oder H-C-$OR^4$ steht, worin $R^4$ ein Wasserstoffatom, einen Alkylrest, einen Alkenylrest, einen gegebenenfalls substituierten Benzylrest, einen CO-$R^5$-Rest oder einen CO-$NR^5$-Rest bedeutet, in dem $R^5$ für einen Alkylrest oder für einen gegebenenfalls substituierten Phenylrest steht, oder deren Salze oder Metallkomplexverbindungen.

EP 0 037 468 A2

Croydon Printing Company Ltd.

# Fungizide Mittel, enthaltend Triazolylderivate und Verfahren zur Bekämpfung von Pilzen mit ihnen

Die vorliegende Erfindung betrifft fungizide Mittel, die Triazolylderivate oder deren Salze oder Metallkomplexverbindungen enthalten sowie Verfahren zur Bekämpfung von Pilzen mit ihnen.

Es ist bekannt, daß ß-Azolylketone, zum Beispiel 1-(4'-Chlorphenyl)-1(1'-2'-4'-triazol-1'-yl)-butanon-3 (DE-OS 26 34 511) fungizide Wirksamkeit besitzen. Die Wirkung dieser Stoffe ist bei niedrigen Aufwandmengen und Konzentrationen nicht befriedigend. Darüberhinaus ist ihre fungitoxische Wirkung mit einer Phytotoxizität verbunden, die in den für die Bekämpfung von Pilzen notwendigen Konzentrationen bisweilen Schädigungen der Kulturpflanzen verursacht. Aus diesen Gründen sind sie zur Bekämpfung von Pilzen nicht für alle Getreidearten und -sorten geeignet.

Es wurde nun gefunden, daß Verbindungen der Formel I

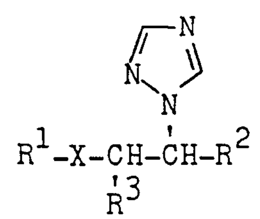

I

in der
$R^1$ und $R^2$ unabhängig voneinander Alkyl mit 1 bis 6 C-Atomen, Naphthyl oder Phenyl bedeuten, wobei der Phenylrest durch Fluor, Chlor, Brom, Alkyl mit 1 bis 4 C-Atomen oder Alkoxy mit 1 bis 4 C-Atomen substituiert sein kann und $R^3$ Alkyl mit 1 bis 4 C-Atomen, Benzyl oder Phenyl bedeutet, wobei der Benzylrest oder der Phenylrest durch Fluor,

Chlor, Brom oder Alkyl mit 1 bis 4 C-Atomen substituiert sein kann und X für C=O oder $H-C-OR^4$ steht, worin $R^4$ ein Wasserstoffatom, einen Alkylrest mit 1 bis 4 C-Atomen, einen Alkenylrest mit 2 bis 4 C-Atomen, einen gegebenenfalls durch Chlor oder Brom substituierten Benzylrest, einen $CO-R^5$-Rest oder einen $CO-NHR^5$-Rest bedeutet, in dem $R^5$ für einen Alkylrest mit 1 bis 4 C-Atomen oder für einen gegebenenfalls durch Chlor, Brom oder Alkyl mit 1 bis 4 C-Atomen substituierten Phenylrest steht, sowie deren Salze und Metallkomplexverbindungen ausgezeichnet wirksam gegen Schadpilze, insbesondere aus der Klasse der Ascomyceten und Basidomyceten sind und eine sehr gute Pflanzenverträglichkeit zeigen.

Die Verbindungen der Formel I besitzen zwei bzw. drei Asymmetriezentren. Aus den bei der Synthese üblicherweise anfallenden Diastereomerengemischen kann man mit bekannten Methoden einheitliche Diastereomerenpaare bzw. einheitliche Enantiomeren erhalten. Diese werden von der vorliegenden Erfindung ebenfalls umfaßt. Als Mittel zur Bekämpfung von Schadpilzen kann man sowohl die einheitlichen Diastereomeren bzw. Enantiomeren wie auch deren bei der Synthese anfallende Gemische verwenden. Bevorzugt werden die letzteren verwendet.

Es wurde weiter gefunden, daß man Triazolylderivate der Formel I dadurch herstellen kann, daß man

a) 1,2,4-Triazol mit α,β-ungesättigten Ketonen der Formel II

$$R^1-\overset{O}{\overset{\|}{C}}-\underset{R^3}{\underset{|}{C}}=CH-R^2 \qquad II$$

in welcher $R^1$, $R^2$ und $R^3$ die oben angegebenen Bedeutungen haben, gegebenenfalls in Gegenwart eines Lösungs- oder Verdünnungsmittels und gegebenenfalls in Gegenwart eines basischen Katalysators umsetzt.

Die α,ß-ungesättigten Ketone der Formel II sind teilweise bekannt wie beispielsweise das 1,2,3-Triphenylpropen-2-on-1, für das in der DE-OS 26 59 293 eine günstige Synthese beschrieben wird. Analog oder nach den bekannten Methoden der gezielten Aldolkondensation (vgl. z.B. T.Mukaiyama, K.Banno und K.Narasaka, J.Am.Chem.Soc. 96 (1974), S. 7503) können die entsprechenden α,ß-ungesättigten Ketone der Formel II hergestellt werden.

Die Umsetzung der α,ß-ungesättigten Ketone mit 1,2,4-Triazol nach dem oben beschriebenen Verfahren zu den ß-Triazolylketonen der Formel III

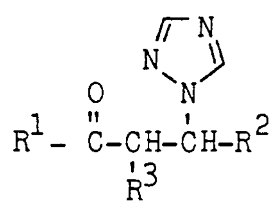

III

in der $R^1$, $R^2$ und $R^3$ die oben genannten Bedeutungen haben, wird zweckmäßig ohne Verdünnungsmittel oder in einem indifferenten Verdünnungsmittel wie beispielsweise Methanol, Ethanol, Isopropanol, Tetrahydrofuran, Dioxan, Toluol, Acetonitril oder Dimethylformamid bei Temperaturen von 0 bis 100°C, vorzugsweise +20 bis +60°C durchgeführt.

Es ist zweckmäßig, einen basischen Katalysator wie z.B. Natriumhydroxid, Kaliumhydroxid, Triethylamin

oder N,N-Dimethylcyclohexylamin dem Reaktionsgemisch zuzugeben.

Es wurde weiter gefunden, daß man Triazolylderivate der Formel I auch erhält, wenn man

b) ß-Triazolylketone der Formel III katalytisch mit Wasserstoff oder mit Hydriden zu den γ-Triazolylalkoholen der Formel IV

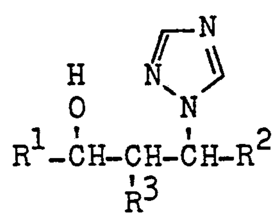

IV

in der $R^1$, $R^2$ und $R^3$ die oben genannten Bedeutungen haben, gegebenenfalls in Gegenwart eines Lösungs- oder Verdünnungsmittels reduziert und

c) γ-Triazolalkohole der Formel IV

α) mit einem Alkylierungsmittel der Formel V umsetzt

$$Hal-R^4 \qquad V$$

worin Hal für Chlor, Brom oder Jod steht und $R^4$ die oben angegebene Bedeutung mit Ausnahme des Wasserstoffs hat oder

ß) mit einem Säurehalogenid der Formel VI umsetzt

$$Hal-\overset{O}{\overset{\|}{C}}-R^5 \qquad VI$$

worin Hal für Chlor oder Brom und $R^5$ für die oben genannten Reste steht oder

γ) mit einem Isocyanat der Formel VII

$$O=C=N-R^5 \qquad VII$$

worin $R^5$ die oben angegebene Bedeutung hat, umsetzt, wobei die Umsetzungen α, ß und γ jeweils gegebenenfalls in Gegenwart eines Lösungs- oder Verdünnungsmittels bei Temperaturen zwischen +10 und +100°C gegebenenfalls in Gegenwart anorganischer und organischer Basen sowie gegebenenfalls in Gegenwart eines Reaktionsbeschleunigers durchgeführt werden und die gemäß Verfahren a) bis c) erhaltenen Verbindungen gegebenenfalls mit Säuren oder Metallsalzen umsetzt.

Geeignete Lösungs- oder Verdünnungsmittel für das Verfahren b) sind beispielsweise Wasser, Methanol, Ethanol, Diethylether, Tetrahydrofuran, Dioxan oder Toluol.

Für die als Reduktionsmittel verwendbaren Hydride seien beispielsweise Natriumborhydrid oder Lithiumaluminiumhydrid genannt.

Zur katalytischen Hydrierung verwendet man Platin- oder Palladiumkatalysatoren auf inerten Trägern und hydriert bei Drucken von 2 bis 50 bar bis keine Wasserstoffaufnahme mehr erfolgt.

Für die unter c) genannten Verfahren eignen sich folgende Lösungs- oder Verdünnungsmittel: Diethylether, Tetrahydrofuran, Dioxan, Cyclohexan, Methylenchlorid, Chloroform, Toluol oder Dimethylformamid.

Für die erwähnten anorganischen oder organischen Basen seien beispielsweise genannt: Alkali- und Erdalkalihydroxide wie Natriumhydroxid, Kaliumhydroxid, Calziumhydroxid, Alkalicarbonate wie Kalium- oder Natriumcarbonat, Alkalihydride wie Natriumhydrid, Alkali- oder Erdalkalialkoholate wie Natriummethylat, Magnesiummethylat oder Natriumisopropylat oder tertiäre Amine wie Trimethylamin, Triethylamin, N,N-Dimethylanilin, N,N-Dicyclohexylamin, N-Methylpiperidin oder Pyridin.

Mit geeigneten Basen wie z.B. Alkalihydrid wie Natriumhydrid oder Alkali- oder Erdalkalialkoholaten wie Natriummethylat können die γ-Triazolylalkohole der Formel IV auch in einer ersten Umsetzung zunächst in ihre Alkoholat-Salze überführt werden und dann als solche zur Reaktion gebracht werden.

Als Reaktionsbeschleuniger für die unter c) genannten Verfahren kommen vorzugsweise Metallhalogenide wie Kaliumjodid, Kronenether, quartäre Ammoniumverbindungen wie Tetrabutylammoniumjodid oder Kombinationen dieser Reaktionsbeschleuniger in Frage.

Die so erhaltenen Verbindungen der Formel I werden nach üblichen Methoden isoliert. Im allgemeinen bedürfen sie keiner weiteren Reinigung, sie können aber nach bekannten Methoden wie Umkristallisation, Extraktion, Destillation oder Chromatographie weiter gereinigt werden.

Die Wirkstoffe der Formel I können auch in Form ihrer Salze, insbesondere in Form der für Pflanzen verträglichen Salze, wie Säureadditionssalze z.B. Hydrochloride, Hydrooxalate oder Hydronitrate verwendet werden.

Falls gewünscht können die Triazolylderivate der Formel I auch nach bekannten Verfahren in ihre Metallkomplexe, insbesondere in die für Pflanzen verträglichen Metallkomplexe, überführt werden. Solche Metallkomplexe erhält man beispielsweise durch Umsetzung eines Triazolylderivates der Formel I mit einem Metallsalz wie einem Salz von Kobalt, Eisen, Nickel, Kupfer, Zink oder Mangan z.B. Kobaltchlorid, Eisen-II-sulfat, Nickelnitrat, Kupfer-II-sulfat, Zinkchlorid, Kupfer-II-chlorid oder Mangan-II-sulfat.

Die folgenden Vorschriften erläutern die Herstellung der Verbindungen:

<u>Vorschrift 1</u>

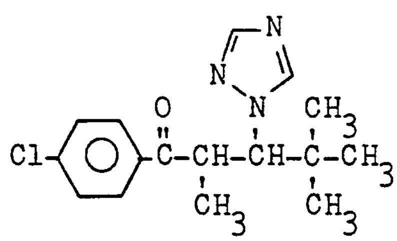 (Nr. 1)

31 g 1-(4'-Chlorphenyl)-2,4,4-trimethylpenten-2-on-1 werden in 250 ml Ethanol mit 27 g 1,2,4-Triazol und 0,2 g Kaliumhydroxid vier Tage lang bei 60°C gerührt. Dann wird das Reaktionsgemisch eingeengt, in Methylenchlorid aufgenommen und viermal mit Wasser gewaschen. Die organische Phase wird getrocknet, eingeengt und an Kieselgel mit Cyclohexan-Essigester-(1 bis 50 % Essigester)-Gemischen getrennt. Man erhält so 12 g 4-Chlorphenyl-(3'-(1",2",4"-triazol-1"-yl)-4',4'-dimethylpentyl-2')-keton als hellgelbes Öl. $n_D^{20}$ = 1,5172

| | C | H | N | Cl |
|---|---|---|---|---|
| berechnet | 62,8 | 6,6 | 13,7 | 11,6 |
| gefunden | 62,7 | 6,2 | 13,2 | 11,2 |

Vorschrift 2

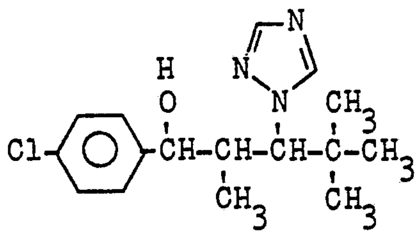

(Nr. 2)

14 g 4-Chlorphenyl-(3'-(1",2",4"-triazol-1"-yl)-4',4'-dimethylpentyl-2'-)-keton werden in 250 ml abs. Methanol gelöst und bei $0^{o}C$ portionsweise mit 2,75 g Natriumborhydrid versetzt. Nach dem Rühren über Nacht wird die Mischung mit konz. Salzsäure angesäuert, anschließend mit Ammoniak neutralisiert und das Reaktionsgemisch zur Trockne eingeengt. Der Rückstand wird in Methylenchlorid gelöst und dreimal mit Wasser gewaschen. Nach dem Trocknen und Einengen der organischen Phase verbleiben 11 g 1-(4'-Chlorphenyl)-2,4,4-trimethyl-3-(1",2",4"-triazol-1"-yl)-pentyl-1-alkohol als klares Öl ($n_D^{20}$ = 1,5350).

| | C | H | N | Cl |
|---|---|---|---|---|
| ber. | 62,6 | 6,9 | 13,7 | 11,5 |
| gef. | 62,1 | 6,6 | 13,3 | 10,9 |

Vorschrift 3

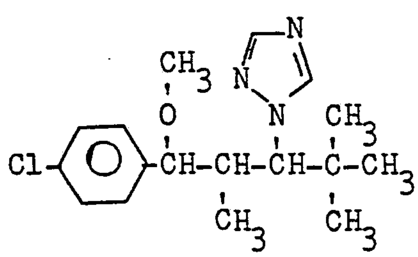

(Nr. 3)

In 150 ml absolut trockenem Tetrahydrofuran (THF) werden 1,7 g Natriumhydrid suspendiert und bei 40°C langsam mit einer Lösung von 10 g 1-(4'-Chlorphenyl)-2,4,4-trimethyl--3-(1",2",4"-triazol-1"-yl)-pentyl-1-alkohol in 50 ml abs. THF versetzt. Nach zweistündigem Rühren bei 50°C wird auf Raumtemperatur abgekühlt und eine Lösung von 7,1 g Methyljodid in 10 ml abs. THF zugetropft. Nach dem Rühren über Nacht wird das überschüssige Natriumhydrid mit Wasser zersetzt. Das Reaktionsgemisch wird mit Schwefelsäure neutralisiert und eingeengt. Der Rückstand wird in Wasser gelöst und zweimal mit Methylenchlorid extrahiert. Die Lösung wird getrocknet und das Lösungsmittel abgedampft. Es verbleiben 9 g Methyl-(1-(4'-chlorphenyl)-2,4,4-trimethyl-3(1",2",4"-triazol-1"-yl)-pentyl-1)-ether als farbloses Öl.

| | C | H | N | Cl |
|---|---|---|---|---|
| ber. | 63,4 | 7,5 | 13,0 | 11,0 |
| gef. | 63,7 | 7,9 | 12,6 | 10,7 |

Vorschrift 4

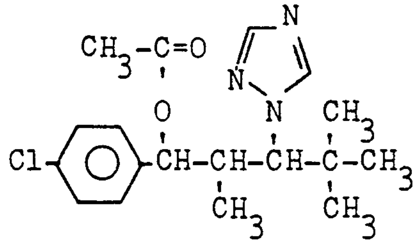

(Nr. 4)

In 200 ml abs. THF werden 15 g 1-(4'-Chlorphenyl)-2,4,4--trimethyl-3-(1",2",4"-triazol-1"-yl)-pentyl-1-alkohol, 1 g Imidazol und 8,5 ml Triethylamin gelöst. Unter Rühren werden 5,6 ml Acetanhydrid zugetropft und anschließend wird für 2 Stunden bei Rückflußtemperatur gekocht und gerührt. Nach dem Abkühlen wird eingeengt, der Rückstand in

Methylenchlorid aufgenommen und dreimal mit Wasser gewaschen. Die organische Phase wird nach dem Trocknen eingeengt und an Kieselgel mit Methylenchlorid als Elutionsmittel gereinigt. Man erhält so 8 g Essigsäure-1-(4'-chlorphenyl)-2,4,4-trimethyl-3(1",2",4"-triazol-1"-yl)-pentyl-1-ester als farbloses Öl ($n_D^{20}$ = 1,5071).

| | C | H | N | Cl |
|---|---|---|---|---|
| ber. | 61,9 | 6,6 | 12,0 | 10,1 |
| gef. | 61,7 | 7,0 | 11,8 | 9,8 |

Analog wurden die folgenden Triazolylderivate der Formel I hergestellt, die durch UR- und NMR-Spektroskopie sowie durch Elementaranalyse charakterisiert wurden.

Tabelle

| Nr. | $R^1$ | X | $R^2$ | $R^3$ | $Fp/n_D^{20}$ |
|---|---|---|---|---|---|
| 5 | $CH_3-$ | C=O | $4-Cl-C_6H_4-$ | $C_6H_5-$ | |
| 6 | $CH_3-$ | C=O | $C_6H_5-$ | $C_6H_5-$ | 184-187 |
| 7 | $CH_3-$ | C=O | $C_6H_5-$ | $4-Cl-C_6H_4-$ | als Hydrochlorid 109-113 |
| 8 | $C_6H_5-$ | HCOH | $C_6H_5-$ | $CH_3-$ | 136-138 |
| 9 | $C_6H_5-$ | C=O | $2-Cl-C_6H_4-$ | $CH_3-$ | 147-150 |
| 10 | $C_6H_5-$ | C=O | $C_6H_5-$ | $CH_3-$ | 111-114 |
| 11 | $C_6H_5$ | C=O | 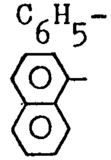 | $CH_3-$ | 179-181 |
| 12 | $C_6H_5-$ | C=O | $n-C_3H_7-$ | $CH_3-$ | Öl |
| 13 | $C_6H_5-$ | C=O | $tert.-C_4H_9-$ | $CH_3-$ | Öl |
| 14 | $4-F-C_6H_4-$ | C=O | $tert.-C_4H_9-$ | $CH_3-$ | 1,518 |
| 15 | $C_6H_5-$ | C=O | $tert.-C_4H_9-$ | $n-C_4H_9-$ | 1,5022 |
| 16 | $C_6H_5-$ | C=O | $i-C_3H_7-$ | $-CH_3$ | Öl |
| 17 | $4-Cl-C_6H_4-$ | C=O | $i-C_3H_7-$ | $-CH_3$ | Öl |
| 18 | $4-Cl-C_6H_4-$ | C=O | $n-C_3H_7-$ | $-CH_3$ | Öl |
| 19 | $4-Cl-C_6H_4-$ | $HC-OCH_2CH_3$ | $tert.-C_4H_9-$ | $-CH_3$ | |
| 20 | $4-Cl-C_6H_4-$ | $HC-OCOCH_2-CH_3$ | $tert.-C_4H_9-$ | $-CH_3$ | 1,5009 |
| 21 | $C_6H_5-$ | C=O | $cyclo-C_6H_{11}-$ | $CH_3-$ | |
| 22 | $4-Cl-C_6H_4-$ | C=O | $cyclo-C_6H_{11}-$ | $CH_3-$ | Harz |

| Nr. | $R^1$ | X | $R^2$ | $R^3$ | $Fp/n_D^{20}$ |
|---|---|---|---|---|---|
| 23 | $C_6H_5-$ | C=O | $C_6H_5-$ | $C_6H_5-$ | |
| 24 | $C_6H_5-$ | HCOH | $C_6H_5-$ | $C_6H_5-$ | |
| 25 | $C_6H_5-$ | $HCO(CO)NHCH_3$ | $C_6H_5-$ | $C_6H_5-$ | |
| 26 | $C_6H_5-$ | C=O | $4-Cl-C_6H_5-$ | $C_6H_5-$ | |
| 27 | $4-Cl-C_6H_4-$ | $HCOCH_2-CH=CH_2$ | tert.-$C_4H_9-$ | $CH_3-$ | |
| 28 | $4-Cl-C_6H_4-$ | $HCOCH_2C_6H_5$ | tert.-$C_4H_9-$ | $CH_3-$ | |
| 29 | $4-Cl-C_6H_4-$ | $HCO(CO)C_6H_5$ | tert.-$C_4H_9-$ | $CH_3-$ | |
| 30 | $4-Cl-C_6H_4-$ | $HCO(CO)NHC_6H_5$ | tert.-$C_4H_9-$ | $CH_3-$ | |
| 31 | $CH_3-$ | C=O | $4-Br-C_6H_4-$ | $C_6H_5-$ | 106-108 |
| 32 | $CH_3-$ | C=O | $2-OCH_3-C_6H_4-$ | $C_6H_5-$ | 176-178 |
| 33 | $CH_3-$ | C=O | $4-OCH_3-C_6H_4-$ | $C_6H_5-$ | 153-155 |
| 34 | $CH_3-$ | C=O | $2,4-Cl_2-C_6H_3-$ | $C_6H_5-$ | 104-106 |

Die Wirkstoffe und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, sowie in Gemüsen wie Gurken, Bohnen oder Kürbisgewächsen.

Die Verbindungen sind insbesondere geeignet zur Bekämpfung folgender Pflanzenkrankheiten:
Erysiphe graminis (echter Mehltau) in Getreide,
Erysiphe cichoriacearum (echter Mehltau) an Kürbisgewächsen,
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helminthosporiumarten an Getreide,
Ustilago-Arten an Getreide und Zuckerrohr,
Rhynchosporium secale an Getreide,
Venturia inaequalis (Apfelschorf).

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die Wirkstoffe können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung oder wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkyl-sulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha. Die neuen Verbindungen können auch im Materialschutz u.a. zur Bekämpfung holzzerstörender Pilze, wie Coniophora puteana und Polystictus versicolor eingesetzt werden. Bei der Anwendung der Wirkstoffe im Materialschutz, z.B. als Fungizide für Anstrichfarben und Weich-Polyvinylchlorid, betragen die Aufwandmengen 0,05 bis 5 Gew.% Wirkstoff, bezogen auf das Gesamtgewicht der zu konservierenden Farben bzw. des mikrozidauszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung der Vorschrift 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung der Vorschrift 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100.000 Gewichtsteilen Wasser erhält man eine wäßrige Dispersion, die 0,02 Gew.% des Wirkstoffs enthält.

III. 20 Gewichtsteile der Verbindung der Vorschrift 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile der Verbindung der Vorschrift 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile der Verbindung Nr. 7 werden mit 3 Gewichtsteilen des Natriumsalzes der Diisobutyl-naphthalin-α-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile der Verbindung Nr. 2 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 3 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

IX. 20 Teile der Verbindung Nr. 4 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,
Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat und
Zinkethylenbisdithiocarbamat,
Tetramethylthiuramidsulfide,
Ammoniak-Komplex von Zink-(N,N-ethylen-bis-dithiocarbamat) und N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und N,N'-Propylen-bis(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Substanzen, wie
N-Trichlormethylthio-tetrahydrophthalimid,
N-(1,1,2,2-Tetrachlorethylthio)-tetrahydrophthalimid,
N-Trichlormethylthio-phthalimid,
2-Heptadecyl-2-imidazolin-acetat,
2,4-Dichlor-6-(o-chloranilino)-s-triazin,
O,O-Diethyl-phthalimidophosphonothioat,

5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-Chlorphenyl)-pyridinmethanol,
1,2-Bis-(3-ethoxycarbonyl-2-thioureido)-benzol,
1,2-Bis-(3-methoxycarbonyl-2-thioureido)-benzol

und weitere Substanzen, wie
Dodecylguanidinacetat,
3-(3-(3,5-Dimethyl-2-oxycyclohexyl)-2-hydroxyethyl)-glutarimid,
Hexachlorbenzol,
N-Dichlorfluormethylthio-N',N'-dimethyl-N-phenyl-schwefelsäurediamid
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Iod-benzoesäure-anilid,
1-(3,4-Dichloranilino)-1-formylamino-2,2,2-trichlorethan,

2,6-Dimethyl-N-tridecyl-morpholin bzw. dessen Salze,
2,6-Dimethyl-N-cyclododecyl-morpholin bzw. dessen Salze,

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Die folgenden Versuche erläutern die fungizide Wirkung der Wirkstoffe. Als bekannter Vergleichswirkstoff A wurde die aus der DE-OS 26 34 511 bekannte Verbindung 1-(4'-Chlorphenyl)-1(1',2',4'-triazol-1'-yl)-butanon-3 verwendet.

Versuch 1
Wirksamkeit gegen Gerstenmehltau

Blätter von in Töpfen gewachsenen Gerstenkeimlingen werden mit wäßrigen Emulsionen aus 80 % (Gew.-%) Wirkstoff und 20 % Emulgiermittel besprüht und nach dem Antrocknen des Spritzbelages mit Oidien (Sporen) des Gerstenmehltaus (Erysiphe graminis var. hordei) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltaupilzentwicklung ermittelt.

Das Ergebnis des Versuches zeigt, daß die Wirkstoffe 1, 2, 3, 6, 7, 9, 10 und 14 eine bessere fungizide Wirkung haben als der Wirkstoff A.

Versuch 2

Blätter von in Töpfen gewachsenen Weizenkeimlingen der Sorte "Jubilar" werden mit wäßrigen Emulsionen aus 80 % (Gew.-%) Wirkstoff und 20 % Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltauentwicklung ermittelt.

Das Ergebnis des Versuches zeigt, daß die Wirkstoffe 1, 2, 3, 4, 6, 7, 8, 9, 10, 14, 17, 20, 31 und 34 eine bessere fungizide Wirkung haben als der Wirkstoff A.

## Patentansprüche

1. Fungizide Mittel, enthaltend ein Triazolylderivat der allgemeinen Formel

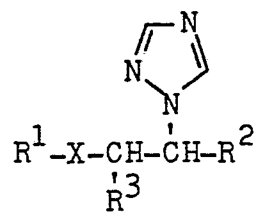

in der
$R^1$ und $R^2$ unabhängig voneinander Alkyl mit 1 bis 6 C-Atomen, Naphthyl oder Phenyl bedeuten, wobei der Phenylrest durch Fluor, Chlor, Brom, Alkyl mit 1 bis 4 C-Atomen oder Alkoxy mit 1 bis 4 C-Atomen substituiert sein kann und $R^3$ Alkyl mit 1 bis 4 C-Atomen, Benzyl oder Phenyl bedeutet, wobei der Benzylrest oder der Phenylrest durch Fluor, Chlor, Brom oder Alkyl mit 1 bis 4 C-Atomen substituiert sein kann und X für C=O oder H-C-$OR^4$ steht, worin $R^4$ ein Wasserstoffatom, einen Alkylrest mit 1 bis 4 C-Atomen, einen Alkenylrest mit 2 bis 4 C-Atomen, einen gegebenenfalls durch Chlor oder Brom substituierten Benzylrest, einen CO-$R^5$-Rest oder einen CO-$NHR^5$-Rest bedeutet, in dem $R^5$ für einen Alkylrest mit 1 bis 4 C-Atomen oder für einen gegebenenfalls durch Chlor, Brom oder Alkyl mit 1 bis 4 C-Atomen substituierten Phenylrest steht, sowie deren Salze und Metallkomplexverbindungen.

2. Verfahren zur Bekämpfung von Pilzen, <u>dadurch gekennzeichnet</u>, daß man ein Triazolylderivat gemäß Anspruch 1 auf diese einwirken läßt.

3. Verfahren zur vorbeugenden Bekämpfung von Pilzen, dadurch gekennzeichnet, daß man mindestens ein Triazolylderivat gemäß Anspruch 1 auf durch Pilzbefall bedrohte Gegenstände einwirken läßt.

4. Fungizid gemäß Anspruch 1, enthaltend 1,2-Diphenyl-1-(1',2',4',triazol-1'-yl)-butanon-3.

5. Fungizid gemäß Anspruch 1, enthaltend 1-Phenyl-1-(1',2', 4'-triazol-1'-yl)-2-(4'-chlor-phenyl)-butanon-3-hydrochlorid.